Europäisches Patentamt

European Patent Office

Office européen des brevets

(11) Publication number: **0 194 496**
A2

# (12) EUROPEAN PATENT APPLICATION

(21) Application number: **86102378.6**

(22) Date of filing: **24.02.86**

(51) Int. Cl.⁴: **G 01 N 33/535**
//G01N33/564

(30) Priority: **08.03.85 JP 46753/85**

(43) Date of publication of application:
**17.09.86 Bulletin 86/38**

(84) Designated Contracting States:
**CH DE FR IT LI NL SE**

(71) Applicant: **SHIONOGI & CO., LTD.**
**12, Dosho-machi 3-chome Higashi-ku**
**Osaka 541(JP)**

(72) Inventor: **Mikawa, Haruki**
**6-16, Oharanokamizatokatsuyama-cho**
**Nishigyo-ku Kyoto-shi(JP)**

(72) Inventor: **Hosoi, Susumu Kotohaitsufushimiinari C-525**
**1-1 Fukakusashimogawara-cho**
**Fushimi-ku Kyoto-shi Kyoto(JP)**

(72) Inventor: **Harada, Shigenori**
**1-6-2-601, Takanodai**
**Suita-shi Osaka(JP)**

(74) Representative: **Hranitzky, Wilhelm Max et al,**
**NOVAPAT - CABINET CHEREAU 5, Place du Molard**
**CH-1204 Genève(CH)**

(54) **Reagents for determining allergen specific IgG antibody.**

(57) Diagnostic aids for determining human IgG antibodies specific for allergens such as mite, egg white, cryptomeria pollen, kamogaya in Japensess name (Dactylis glomerata) pollen, mugwort pollen, fungi and cat's scurf, causing allergic diseases such as exogenous bronchial asthma, atopic dermatitis, immune complex diseases, etc., comprising (a) enzym-labelled anti-IgG reagent, (b) colouring agent, (c) substrate reagent, (d) reaction-terminating agent, (e) buffering agent, (f) washing agent and (g) diluting agent.

EP 0 194 496 A2

REAGENTS FOR DETERMINING ALLERGEN SPECIFIC IgG ANTIBODY.

Allergic diseases caused by antigen-antibody reactions now can be classified diagnostically by search and identification of the causal matter (allergen), upon which it is possible to make a course for curative treatment. The specific IgG test reagents of the present invention can be used as diagnostic agents in identification of allergens in allergic diseases such as exogenous bronchial asthma, atopic dermatitis, immune complex disease, and the like, or as monitors for immunologically tracing clinical progress of patients.

For these several decades, it has been recognized that the determination of immunoglobulin G (IgG) antibody against human serum allergen is very important [J.W. Younginger et al., J.Clin. Invest., 52, 1268 (1973); A.K. Sobotka et al., J.Immunol., 117, 84 (1976)]. Recently, a radioallergosorbent test (hereinafter referred to as RAST) has been utilized in determination of IgE antibody specific to human serum allergen [N.F. Adkinson, Jr., American Society for Microbiology, Washington, D.C., p. 590-602 (1976)]. The determination of human allergen-specific IgG antibody, however, has not yet been succeeded by means of RAST except only one report [Shimizu et al., J.Immunol.Methods, 19, 317 (1978)]. The reason of unsuccessful result is assumed that in RAST the binding level of non-specific antibody in normal serum is greater than that of specific antibody. Hamilton and his co-workers have succeeded in decreasing the non-specific binding by using $^{125}$I-protein A isolated from Staphylococcus aureus in place of rabbit's anti-human IgG antibody [J.Immunol., 122, 1073 (1979)]. Protein A isolated from Staphylococcus aureus preferentially binds to the Fc portion of human IgG subclass 1, 2 and 4 but not to human $IgG_3$. Although Protein A binds to some immunoglobulin A (hereinafter referred to as IgA) subclass 1 and 2, there seems no simple relationship between IgA subclass and the binding ability of protein A [Brunda et al., J.Immunol., 123, 1457 (1979)]. Johansson and Inganas have reported that immunoglobulin E (herein-after referred to as IgE) isolated from a serum which contains polyclonal IgE has high affinity with protein A but the binding site different from that of IgG is not the Fc portion [Immunological Rev., 41, 248 (1978)].

In view of the above situation, it has been greatly expected that a new method for determining human allergen-specific IgG antibody is developed.

The present inventors have been succeeded in producing in the mouse abdominal cavity a large quantity of monoclonal antibody which binds with a uniform affinity to the Fc site of human IgG [Japanese Patent Application no. 87190/1984].

Diagnostic aids for determining human serum allergens such as mite, egg white, cryptomeria pollen, kamogaya in Japensess name (Dactylis glomerata) pollen, mugwort pollen, fungi and cat's scurf, causing allergic diseases such as exogenous bronchial asthma, atopic dermatitis, immune complex diseases, etc., comprising (a) enzyme-labelled anti-IgG reagent, (b) colouring agent, (c) substrate reagent, (d) reaction-terminating agent, (e) buffering agent, (f) washing agent and (g) diluting agent.

Fig. 1 shows reference curves which were drawn by measuring D.P. specific IgG antibody with an enzyme-labelled IgG (0.05 $\mu$ 1/ml); the ordinate indicates the absorbance and the abscissa does the titer of D.P. specific IgG antibody. The marks $\bigcirc$, $\bullet$ and $\triangle$ indicate that the content of anti-hIgG added to a prefixed amount of enzyme-labelled anti-IgG is 0, 0.0025 $\mu$ g/ml and 0.0075 $\mu$ g/ml, respectively.

Fig. 2 shows a reference curve drawn with the reference sera A to F; the ordinate indicates the absorbance and the abscissa does the titer of D.P. specific IgG antibody.

Fig. 3 shows a dilution curve with a positive serum prepared by egg white as allergen; the ordinate indicates the absor-bance and the abscissa does the dilution factor of positive serum.

Fig. 4 shows a relationship between the absorbance (ordinate) and the serum dilution rate (abscissa) which was drawn by measuring the D.P. specific IgG antibody with either a commercially available polyclonal antibody or the monoclonal antibody of the present invention.

Fig. 5 shows a relationship between the absorbance (ordinate) and the disc number (abscissa) which was drawn by measuring the D.P. specific IgG antibody with either a D.P. specific allergen disc or an egg white allergen disc.

Fig. 6 shows a relationship between the amount of D.P.specific IgG antibody (ordinate) and the total IgG level (abscissa).

Fig. 7 shows a relation between the absorbance of the reference serum and of the diluted sample serum (ordinate) and the antibody titer (U/ml) or serum dilution rate (abscissa).

Fig. 8 shows a relation between the absorbance of the diluted reference serum (ordinate) and the antibody titer (abscissa); the arrow indicates the limitation of sensitivity in measurement.

In identifying the allergen of some allergic diseases, it is considered that the reaction medium of an allergen-immobilized solid phase with a serum sample to be tested may exist in the following three conditions.

(a) Condition in which the immobilized allergen normally reacts with the allergen-specific IgG antibody.

(b) Condition in which IgG or IgG antibody does not react with the immobilized allergen but they are adsorbed non-specifically on the solid phase.

(c) Condition in which IgG is remaining in the solution outside of the solid phase medium.

In order to determining the IgG antibody in high specificity, it is desirable for the anti-human IgG antibody to satisfy the following conditions since its character is important factor.

(1) Lower affinity with the IgG which is non-specifically adsorbed as mentioned in (b).

(2) Lower affinity with the IgG which is in free state in a solution as mentioned in (c).

(3) Very high affinity with the IgG which binds to the immobilized allergen as mentioned in (a).

The reagents for determining allergen specific IgG antibody of the present invention satisfy the aforementioned conditions (1) to (3), wherein a monoclonal anti-human IgG antibody suitable for determination of IgG antibody has been employed. Accordingly, it is possible in the present invention to exactly follow up the IgG antibody binding to allergen as well as blocking antibody and reagin-like IgG antibody.

The reagents for determining allergen specific IgG antibody provided by the present invention are used as diagnostic aids based on enzyme immunoassay (EIA), which contain an enzyme-labelled, particularly, peroxidase-labelled monoclonal anti-human IgG antibody (hereinafter referred to as anti-hIgG) as main component and comprise the following components.

(a) Enzyme-labelled anti-IgG reagent

(b) Colouring agent

(c) Substrate reagent

(d) Reaction-terminating agent

(e) Buffering agent

(f) Washing agent and

(g) Diluting agent

In the aforementioned components, the enzyme-labelled anti-IgG reagent contains enzyme-labelled anti-hIgG as main component and comprises the following components.

(a) Human serum albumin

(b) Sodium dihydrogenphosphate

(c) Disodium hydrogenphosphate

(d) Sodium chloride

(e) Anti-hIgG labelled with peroxidase

(f) Preservative                       small amount

(g) Acid and/or alkali                 proper amount

Each component can be explained in more detail as follows.

(1) Monoclonal anti-human IgG antibody

The anti-hIgG used in the preparation of enzyme-labelled anti-IgG of the present invention is a monoclonal antibody HG2-25 which was prepared by the present inventors by cell fusion [Jap. Patent Application no. 87190/84] and shows the following characeristics.

(a) To recognize the $CH_2$ domain of human IgG.

(b) To have high binding ability to $IgG_1$, $IgG_2$, $IgG_3$ and $IgG_4$ belonging to human IgG subclass.

(c) To selectively bind to specific IgG antibody binding to allergen in comparison with immobilized non-specific IgG antibody, while the binding ability to human IgG free in the solution is very weak.

Example of the preparation of monoclonal antibody HG2-25 is disclosed below as "Preparation".

(2) Enzyme-Labelled anti-hIgG

Anti-hIgG is labelled by binding to a peroxidase, particularly, horseradish peroxidase (hereinafter abbreviated to HRP). The binding of anti-hIgG to HRP may be carried out in a known method; for example, HRP is allowed to react with a periodate to form partially aldehyde group, to which is then bound a polypeptide anti-hIgG; the reduction with sodium or potassium borohydride or its equivalent reagents affords stable enzyme-labelled anti-hIgG complex.

(3) Enzyme-Labelled anti-IgG Reagent

The enzyme-labelled anti-hIgG complex prepared in the above item (2) may be preserved in a conventional phosphate buffered physiological saline (PBS) to which is added serum albumin. If necessary, sodium azide or Kathon CG (Rohm and Haas) as preservative or stabilizer may be added. Representative of components constituting are as mentioned above.

The reagents of the present invention may be preserved in a condition of pH 7.0 - 8.0, preferably pH 7.3 - 7.6, more preferably pH 7.4, and a temperature of room temperature or lower, preferably 2 - 8 °C. The pH may be adjusted with addition of an acid such as hydrochloric acid or sulfuric acid like inorganic acid and of an alkali such as sodium hydroxide or sodium carbonate like inorganic base.

(4) Colouring Agent

Compounds which are coloured by oxidation with hydrogen peroxide ($H_2O_2$) catalyzed by a peroxidase, for example, leuco crystal violet (LCV; K&K Laboratory), 2,2'-azino-di(3-ethylbenzthiazoline-6-sulfonic acid (ABTS), may be employed. For example, when ABTS is used, the maximum wave length in visible absorption spectrum shifts from 350 nm to 415 nm. The amount of the colouring agent to be used is in about 0.1 - 1.0 mg per operation, preferably 0.4 - 0.8 mg.

(5) Substrate Reagent

As for a substrate in the enzyme reaction, 0.05 - 0.6% hydrogen peroxide solution, preferably 0.08 - 0.5% one, which contains 0.05 - 0.2% phosphoric acid as stabilizer, may be used in an amount of about 1.0 - 3.0 ml, preferably 2.0 - 2.5 ml per operation.

(6) Reaction Terminating Agent

In order to stop the enzymatic reaction after lapse of a prefixed time, 0.1 - 0.5% sodium azide solution is added as a reaction-terminating agent. More preferably, 0.2 - 0.3% sodium azide solution may be used in an amount of about 1 - 3 ml, preferably about 1.5 - 2.5 ml per operation.

(7) Buffering Agent

The buffering agent is used in dissolving the aforementioned colouring agent or in diluting the aforementioned substrate reagent or reaction terminating agent. The buffering agent includes 0.2 - 0.6M solution of citric acid (3.0 - 8.0%) and a citrate (5.0 -10.0%) (e.g., sodium citrate, potassium citrate). If required, sodium azide or Kathon CG may be added as a stabilizer.

The buffering agent, in using, is once diluted with water to about 50-fold volume, by which the aforementioned reagents are dissolved or diluted. For example, the aforementioned colouring agent is dissolved in the diluted buffer solution, and the substrate reagent is added thereto to give a substrate solution. The addition of the above 50-fold buffer solution to the reaction-terminating agent affords a solution for the reaction termination.

(8) Washing Agent

The washing agent is used in washing of the reaction medium after termination of the antigen-antibody reaction or enzymatic reaction in determination of IgG antibody, and it may usually be used after 10 - 50-fold dilution. The agent which is a buffer of phosphate buffered physiological saline type comprises 15 - 35% of sodium chloride, phosphoric acid salt (e.g., 2.0 - 7.0% sodium dihydrogenphosphate), as well as stabilizer, 1.0 - 3.0% of surfactant (e.g., Tween 20) and 0.05 - 3.0% of alkali metal hydroxide, and it may be used at pH 7.0 - 7.8, preferably at pH 7.2 - 7.6.

(9) Diluting Agent

The agent is used in dilution of samples collected from a person to be examined, particularly serum. The agent is 0.1 - 0.3M phosphate buffered physiological saline (6 - 12% of sodium chloride, 1.0 - 3.0% sodium dihydrogenphosphate, 0.1 - 1.0% alkali metal hydroxide, and a small amount of preservative) containing about 0.05 - 3.0%, preferably about 1 - 2% human serum albumin, and it may be diluted with water to about 8 - 12-fold, preferably about 10-fold volume before using. If necessary, a stabilizer may be added.

A group of the aforementioned reagents is used as an enzyme tracer kit in determination of the serum IgG antibody titer together with a standard kit for drawing every allergen standard curve and an allergen kit which is prepared by fixing allergen on a proper carrier.

The following variety of allergens can be detected by the reagent of the present invention: mite, egg white, pollen of cryptomeria (Cryptomeria japonica), pollen of kamogaya in Japanese name (Dactylis glomerata), pollen of mugwort (Artenusua ubduca), fungi [e.g., Altelnaria, Candida], cat's scurf. In the allergen kit, the above allergens may be fixed on an appropriate carrier, for example, beads, plates, sheets or tubes of glass or synthetic resin. The commercially available allergen-holding paper disc [made by Pharmacia Diagnostic Co.] may be employed.

Procedure for Measurement

(a) Reaction vessels, for example, glass or plastic test tubes, are provided. Two test tubes for the blank test, some pairs for the reference (ordinarily, 10 - 14 tubes), and two for the sample to be tested (ordinarily, serum), may be used. The fixed allergen is placed in each tube (the allergen may be fixed on the inner surface of the tube).

(b) Reference serum (normally prepared by 6 repetition of 2-fold dilutions) or the sample to be tested is placed in the tubes and allowed to stand at room temperature with shaking or stirring.

(c) The reference sample and the sample to be tested are removed by suction and the fixed allergen remaining is washed with the washing solution.

(d) The enzyme-labelled anti-IgG solution is added to the tubes and stirred at room temperature for a period of 10 - 30 hours, preferably 16 - 20 hours.

(e) The liquid portion in the tubes is removed by suction, and the tubes are washed in the same manner as in (c).

(f) The substrate solution is added to the tubes and stirred at room temperature for 10 - 60 minutes. The operation for blank test may be started at this step.

(g) The reaction-terminating solution is added, and the mixture is stirred well at room temperature for about 30 minutes.

(h) The absorbance of the reaction mixture in each test tube is measured at the prefixed wave length [for example, when ABTS is used as colouring agent, the absorbance is measured at 415 nm.].

The present invention is explained in more detail by the following examples and reference examples, which are not intended to restrict the present invention.

Example 1

Enzyme labelling of anti-hIgG

To a solution of 20 mg of horseradish peroxidase (HRP; Sigma Chemical Co.) dissolved in 5 ml of 0.01M acetate buffer (pH 4.5) is added a solution of 5 mg of sodium periodate in 0.25 ml of 0.01M acetate buffer, and the mixture is incubated at room temperature for 20 minutes. The mixture is then passed through a column of Sephadex G-25 (1.5 x 20 cm) and eluted with 0.001M acetate buffer (pH 4.5) to give about 7 ml of eluate of activated HRP. To this fraction is added a solution of 25 mg anti-hIgG in 5 ml of 1M carbonate buffer (pH 9.5), and the mixture is incubated at room temperature for 2 hours. Then, 2 mg of sodium borohydride (NaBH$_4$) is added, and the mixture is incubated at 4°C for 2 hours, applied on a column of Sephadex G-25 (1.5 x 20 cm), and eluted with 0.01M phosphate-buffered physiological saline (hereinafter abbreviated to as PBS)(pH 7.4). The resulting conjugate fractions are condensed to 5 ml, which is applied on a column of Sephacryl S-200 (1.5 x 90 cm), and eluted with 0.01M PBS (pH 7.4). The eluate is condensed to 5 ml, applied on a column of Sepharose 6B (1.5 x 60 cm), and eluted with 0.01M PBS (pH 7.4). The result-ing non-aggregated fractions are condensed, and the condensate is diluted with 0.01M PBS (pH 7.4) containing 0.1% of human serum albumin so as to be 0.156 $\mu$g/ml content; this is used in prepara-tion of the enzyme-labelled anti-IgG reagent.

Confirmation of Enzyme-Labelled anti-hIgG

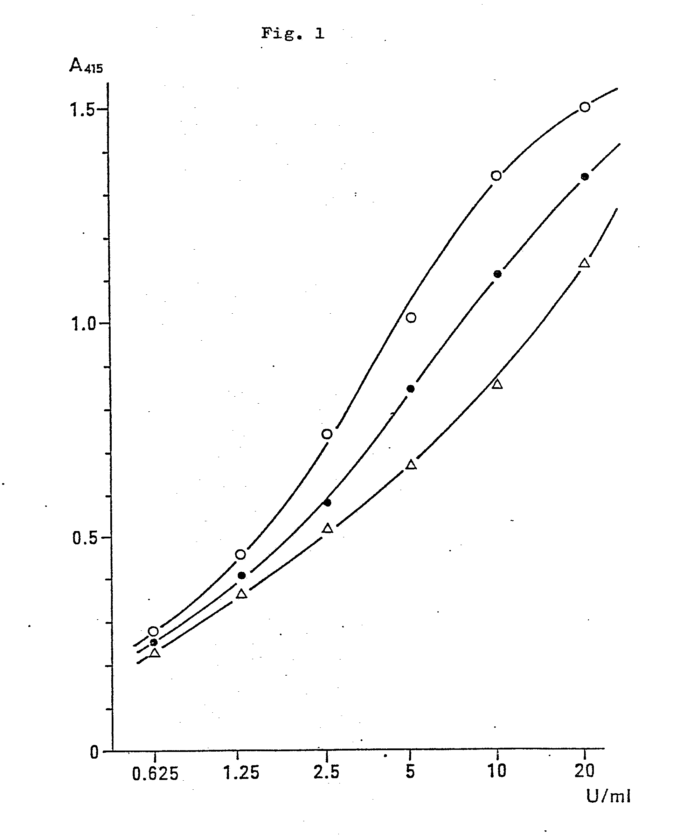

To 0.05 $\mu$g/ml of enzyme-labelled anti-IgG solution is added anti-hIgG in an amount of 0, 0.025 or 0.075 $\mu$g/ml, and standard curves are drawn with respective sera containing D.P. specific IgG antibody. Figure 1 shows the result.

In this procedure, the solution is coloured by the enzyme reaction of HRP, and the decrease of the absorbance and the dose-response are confirmed with increasing to the amount of anti-hIgG added to the enzyme-labelled anti-IgG. This means that the anti-hIgG binds to HRP.

Example 2

Preparation of Reagents

(a) Enzyme-Labelled anti-IgG Reagent

In 12 liter of water are dissolved 15.0 g of human serum albumin, 3.67 g of sodium dihydrogenphosphate dihydrate, 45.33 g of disodium hydrogenphosphate dodecahydrate, 135 g of sodium chloride, 0.825 mg of HRP-labelled anti-hIgG, and 9.0 g of preservative (Kathon CG), and the mixture is adjusted at pH 7.4 with addition of sodium hydroxide or hydrochloric acid and distributed into 550 vials. One vial is used as one kit, which can be used in 100 tests.

(b) Colouring Agent

ABTS 38.625 g is distributed in 550 vials, to which is added purified water in an amount of 31 ml/vial, and lyophilized (one vial can be used in 100 tests).

(c) Substrate Reagent

30% Hydrogen peroxide (5.60 g) and phosphoric acid (1.5 ml) are distributed into 550 vials, to which water is added to make 2.5 ml/vial.

(d) Reaction-Terminating Agent

Sodium azide 2.778 g is distributed into 550 vials, to which is added purified water to make 2 ml/vial (one vial can be used in 100 tests).

(e) Buffering Agent

Citric acid (153.98 g), trisodium citrate (217.78 g) and stabilizer (Kathon CG)(8.42 g) are distributed into 550 vials, to which is added purified water to make 5 ml/vial, of which the pH is adjusted with sodium hydroxide and/or hydrochloric acid (one vial can be used in 100 tests).

(f) Washing Agent

Tween-20 (427.63 g), sodium dihydrogenphosphate dihydrate (1334.3 g), sodium hydroxide (301.0 g), sodium chloride (7650 g) and stabilizer (Kathon CG) are distributed into 550 vials, to which is added purified water to make 60 ml/vial (one vial can be used in 100 tests).

(g) Diluting Agent

Human serum albumin (60.5 g), sodium chloride (544.5 g), sodium dihydrogenphosphate dihydrate (94.38 g), sodium hydroxide (25.0 g) and stabilizer (Kathon CG)(36.3 g) are distributed into 550 vials, to which is added purified water to make 10 ml/vial (one vial can be used in 100 tests).

Example 3

Determination of D.P. Allergen IgG Antibody

(1) Preparation of D.P. Allergen

Mites (Dermatophagoides pteronyssinus) are incubated in a medium containing dry yeast and dry fish meal (1 : 1) at 25°C and 75% humidity. The grown mites are suspended on a saturated aqueous solution of sodium chloride, collected and lyophilized. The lyophilized mites are dispersed homogeneously into 0.01M PBS (pH 7.4) at 4°C and applied to centrifugation (30,000 x g) at 4°C for 1 hour. The supernatant is filtered through a millipore filter (0.22 $\mu$m) to give a crude mite extract.

(2) Fixation of D.P. Allergen (Preparation of Allergen Beads)

To polystyrene beads (diameter; 1/4 inch) is added 0.01M PBS (pH 7.4) at a rate of 0.2 ml/bead. The above-prepared mite extract is made to 25 $\mu$g/ml concentration, added to the bead mixture, and incubated at 37°C overnight. The solution is removed, and the residual beads are washed 3 times with 0.01M PBS (pH 7.4), to which is added 0.01M PBS (pH 7.4) containing 1% human serum albumin, and incubated at 37°C for 1 hour. The liquid portion is removed, and the residual beads are washed 3 times with 0.01M PBS (pH 7.4), to which is added 0.01M PBS (pH 7.4) containing 1% human serum albumin, and incubated at 37°C for 3 hours. Removal of the liquid portion gives D.P. allergen-fixed beads.

(3) Preparation of Reagents Immediately Before Using

(a) Buffer Solution

One vial of the buffering agent (5 ml) is added to 250 ml of purified water and stirred well.

(b) Diluting Solution

One vial of the diluting agent (10 ml) is added to 100 ml of purified water and stirred well.

(c) Washing Solution

One vial of the washing agent (60 ml) is added to 1,500 ml of purified water and stirred well.

(d) Substrate Solution

The buffer solution is added to the colouring agent in an amount of 30 ml per vial, to which is added 2 ml of the substrate reagent and stirred well.

(e) Reaction-Terminating Solution

One vial of the reaction-terminating agent (2 ml) is added to 200 ml of the buffer solution.

(4) Preparation of Reference Serum

A patient serum containing D.P. allergen-specific IgG antibody in high content (antibody titer: 1,000 U/ml) is diluted with 0.1% human serum albumin solution to give a D.P. reference serum.

Reference serum A (antibody titer: 20 U/ml)

Reference serum B (antibody titer: 10 U/ml)

Reference serum C (antibody titer: 4 U/ml)

Reference serum D (antibody titer: 2 U/ml)

Reference serum E (antibody titer: 1 U/ml)

Reference serum F (antibody titer: 0.4 U/ml)

(5) Preparation of Sample

To 20 $\mu$l of patient serum is added 1 ml of the diluting solution and stirred well to give a 51-fold dilution solution.

(6) Steps of Procedure

(a) In plastic test tubes of about 1 cm in inside diameter, two for blank test, twelve for reference sera A to F (every two for each serum) and every two for the sample to be tested is placed every one piece of D.P. allergen-fixed bead.

(b) Reference sera A to F and samples to be tested, every 200 ml, are placed in the aforementioned respective test tubes and the tubes are rotated by an inclined rotator (30 - 35 rotations per minute) at room temperature for 3 hours.

(c) Reference sera and the samples to be tested are removed by suction, and the beads are washed 3 times with 2 ml of washing solution.

(d) Enzyme-labelled anti-IgG solution (200 ml) is added to each test tube and rotated by an inclined rotator (30 - 35 rotations per minute) at room temperature for a period of 16 - 20 hours.

(e) The anti-IgG solution is removed by suction, and the same washing operation as in (c) is applied to the beads.

(f)   The substrate solution, every 300 $\mu$l, is added to all of the tubes including those for blank test, and rotated by an inclined rotator at room temperature for 30 minutes.

(g)   The reaction-terminating solution, 2 ml each, is added to each test tube and stirred well by a vortex mixer.

(h)   The absorbance of the reaction mixture in each tube is measured at 415 nm.

(i)   The values which are obtained by subtraction of the absorbance of the blank from that of the reference serum A to F are plotted on a semi-logarithmic plotting paper in order to draw a reference curve, from which the D.P. allergen IgG antibody titer of the sample to be tested is determined in terms of the absorbance of the sample from which that of the blank has been subtracted.

(7)   Result

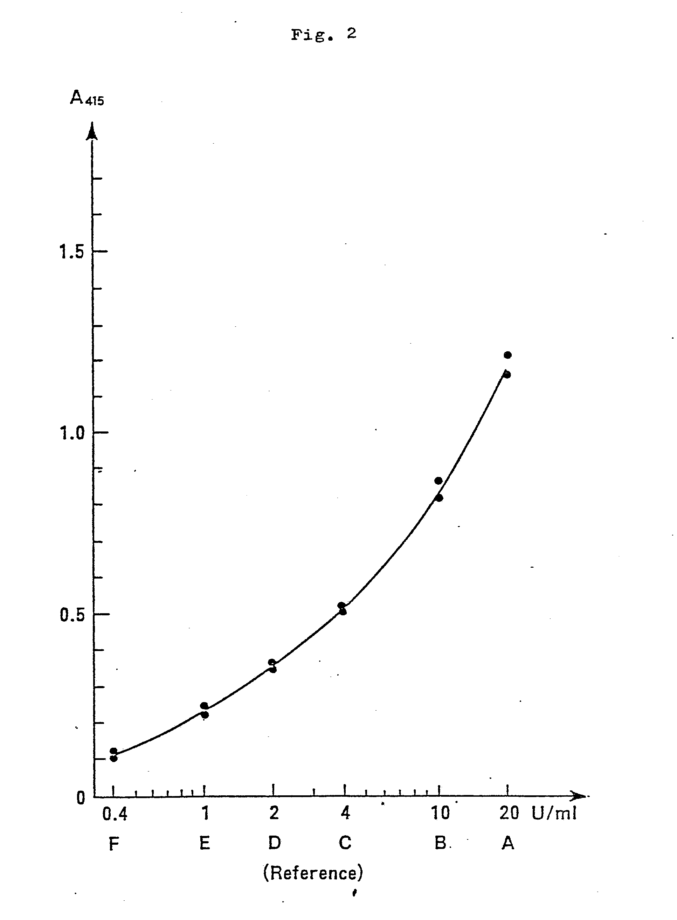

Table 1 shows the absorbance determined and the measured values of the samples (sera of 3 patients under treatment for atopic allergy), and Fig. 2 does the reference curve.

0194496

Table 1

| No. | Reference/Sample | Absorbance | Mean Value * | Measured Value |
|---|---|---|---|---|
| 1 | Blank | 0.008 | 0.007 | ———— |
| 2 | | 0.006 | | |
| 3 | Reference A | 1.205 | 1.182 | ———— |
| 4 | | 1.159 | | |
| 5 | Reference B | 0.853 | 0.831 | ———— |
| 6 | | 0.809 | | |
| 7 | Reference C | 0.522 | 0.516 | ———— |
| 8 | | 0.510 | | |
| 9 | Reference D | 0.361 | 0.355 | ———— |
| 10 | | 0.348 | | |
| 11 | Reference E | 0.236 | 0.228 | ———— |
| 12 | | 0.219 | | |
| 13 | Reference F | 0.123 | 0.117 | ———— |
| 14 | | 0.111 | | |
| 15 | Sample (1) | 0.831 | 0.821 | 495 |
| 16 | | 0.824 | | |
| 17 | Sample (2) | 0.446 | 0.432 | 142.5 |
| 18 | | 0.431 | | |
| 19 | Sample (3) | 0.625 | 0.637 | 305 |
| 20 | | 0.663 | | |

*Mean Value: the blank has been subtracted from the actual absorbance in References A to F and Samples.

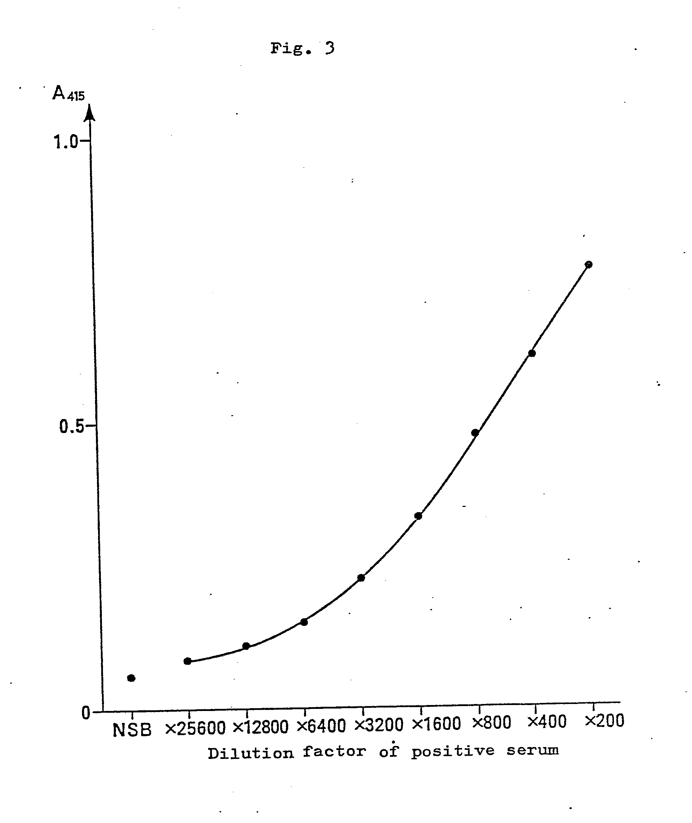

Another test was made with the egg white-fixed beads and the egg white-specific IgG antibody according to the same manner as mentioned above; Fig. 3 shows a dilution curve. In Fig. 3, the NSB (non-specific binding) value was determined by measuring with the diluting solution.

Reference Example

(1)  Preparation of Anti-human IgG Antibody-Producing Hybridoma

BALB/C mouse (8 - 10 weeks of age) was immunized by intraperitoneal administration of 200 $\mu$ g (100 $\mu$ l of physiological saline) of human serum IgG together with 100 $\mu$ l of Freund's complete adjuvant. After lapse of 21 days, an additional 20 $\mu$ g of human serum IgG was administered intraperitoneally as alum precipitate. After lapse of 3 days, the spleen was cut out, dispersed into a RPMI medium to give a cell suspension, 10 ml of which containing $10^8$ spleen cells was mixed with a suspension of 5 x $10^7$ NS-1 myeloma cells in 10 ml of RPMI medium. The mixture was centrifuged at 450 g for 10 minutes, and the supernatant was removed. An RPMI medium (RPMI-1640)(1 ml) containing 50% of PEG 4000 (Merck & Co.) was added thereto with slow stirring, and the mixture was then stirred for 1 minute. An additional 1 ml of the RPMI solution was added thereto over 1 minute. After addition of an additional 1 ml of the solution, an additional 7 ml of the RPMI solution was added over about 3 minutes, and the mixture was centrifuged at 400 g for 10 minutes. After removal of the supernatant, the resulting cells were dispersed into 20 ml of RPMI-1640 medium containing 15% of foetal calf serum (hereinafter referred to as FCS), inoculated on two tissue culture plates (Cohning Co.; 96 wells) at a rate of 100 $\mu$ l/well, and incubated at 37°C under 7% carbon dioxide.

After lapse of 1, 2, 3, 5 and 8 days, the half volume of each medium was changed with a HAT medium (RPMI-1640 medium to which 10% FCS, 4 x $10^{-7}$M aminopterin, 1.6 x $10^{-5}$M thymidine and 1 x $10^{-4}$M hypoxanthine were added), and the incubation was continued. The days 11, 13 and 14, the half of the medium was changed with a HT medium (RPMI-1640 medium to which 10% FCS, 1.6 x $10^{-5}$M thymidine and 1 x $10^{-4}$M hypoxanthine were added).

After lapse of 16 days from the beginning of the fusion, colonies of hybridoma appeared from all of the wells. The hybridoma was incubated on an RPMI-1640 medium containing 15% FCS, and occurrence of the specific antibody in the supernatant of the cultured medium was checked.

The supernatant (5 $\mu$ l) as sample and a phosphate-buffered saline (hereinafter referred to as PBS)(50 $\mu$ l) containing 10% FCS were placed in the respective wells of U-bottom type tissue culture plate, to which was then added 50 $\mu$ l of 1% suspension of sheep red blood cell sensitized with human IgG, and the mixture was slowly stirred. After stationary standing at room temperature for 2 hours, occurrence of hemagglutination was checked. Table 2 shows the result.

Table 2

| Total Well Number | Colony-Appeared Well Number | Antibody-Producing Wells |
|---|---|---|
| 192 | 192 | 72 |

(2)   Cloning of Anti-human IgG Antibody-Producing Cell Line

The anti-human IgG antibody-producing hybridoma prepared in (1) was suspended into an RPMI-1640 medium containing 15% FCS at a rate of 5 cells/ml, and the suspension was inoculated on 40 wells of tissue culture plate (Cohning Co.; 96 wells) so as to be 100 $\mu$ l/well.   The suspension is then diluted so as to be 1 cell/ml and inoculated on 32 wells; it was further diluted to 0.5 cell/ml and inoculated on 24 wells.   After 5 days, 100 $\mu$ l of RPMI-1640 medium containing 15% FCS was added to each well.   For example, in case of clone HG2-25, the hybridoma clone appeared in two of 24 wells containing 0.5 cell/ml and in four of 32 wells containing 1 cell/ml.   Occurrence of the antibody production was checked on the supernatant of each clone culture.

[Result of Test]

The antibody production was recognized in all of the clones.

(3)   Production of Anti-Human IgG Antibody

Into the abdominal cavity of BALB/C mice to whom 0.5 ml of pristene had intraperitoneally been administered was inoculated a suspension of 5 x 10⁶ anti-human IgG antibody-producing hybridoma cells prepared in the above step in 0.5 ml physiological saline. After lapse of 3 weeks, enlargement of the abdomen was observed, and the ascites was collected by an injector and centrifuged for elimination of the cells.

(4) Isolation and Purification of Anti-human IgG Antibody

The ascites collected in the previous step was salted out with 45% saturated aqueous solution of ammonium sulfate, and the resulting precipitate was dissolved in 0.1M phosphate buffer (pH 8.0) and dialyzed in the same buffer. The gamma fraction salted out (20 mg) was dissolved in 2 ml of 0.1M phosphate buffer(pH 8.0) and adsorbed on a column (1.6 x 5 cm) of protein A-Sepharose [Pharmacia AB]. Impurities were first eluted with about 50 ml of 0.1M phosphate buffer (pH 8.0), and then the column was eluted with about 100 ml of 0.1M citrate buffer (pH 5.0) to give the monoclonal anti-human IgG antibody HG2-25.

c. Effect of the Invention

(1) The monoclonal antibody anti-hIgG has very high specificity, by which can be detected a specific IgG antibody which cannot be detected by polyclonal anti-human IgG antibody. The following experiment shows that the said monoclonal antibody has excellent specificity.

Procedure

A serum containing 5 U/ml IgG antibody specific to bee venom was diluted with a diluting solution containing 0.5% normal human serum in order to prepare a standard solution containing 0.1, 0.2, 0.5, 1.0, 2.0 or 5.0 U/ml of specific antibody. Each standard solution was allowed to react with a bee venom-allergen disc at room temperature for 3 hours. After washing, the disc was allowed to react with an optimum concentration of $^{125}$I-labelled monoclonal anti-hIgG antibody or with a commercially available polyclonal anti-human IgG antibody (Pharmacia AB). After washing, the radioactivity was counted, and standard curves were drawn.

Result

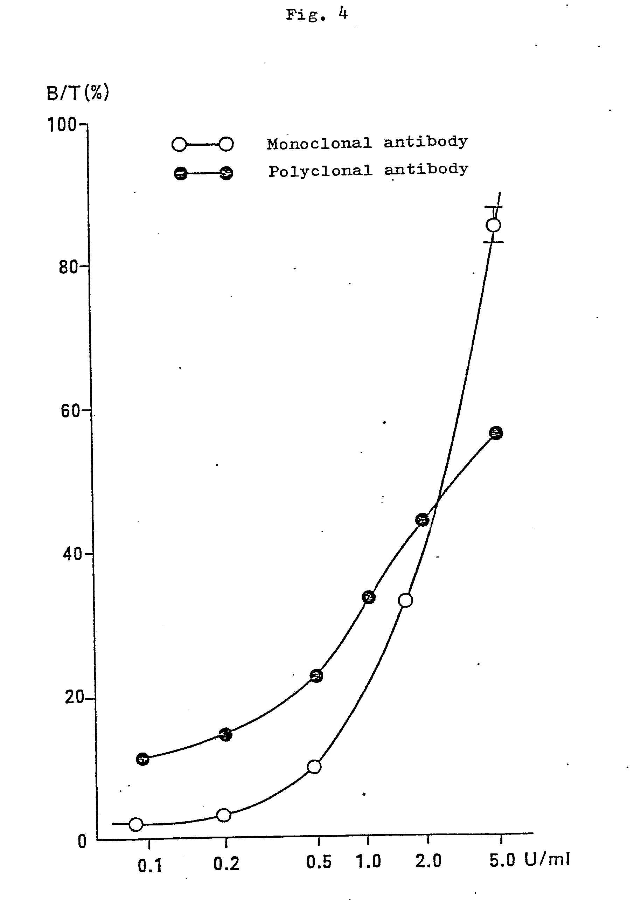

As shown in Fig 4, the monoclonal anti-hIgG antibody has a very low binding rate at 0.1 U/ml, which indicates that the non-specific binding rate is low. On the other hand, the binding rate at the maximum concentration is higher than that of polyclonal antibody, which indicates that the monoclonal antibody has a high specificity.

(2) The anti-hIgG of the present invention exhibits a high allergen specificity and binds specifically to the aimed allergen-binding anti-IgG antibody.

Procedure

A 200-fold diluted standard serum (100 $\mu$ l) containing D.P. specific IgG antibody was allowed to react with a D.P. allergen disc or with an egg white allergen disc at room temperature with shaking for 3 hours, and the D.P. allergen-specific IgG antibody remaining in 50 ml of the reaction mixture was determined.

Result

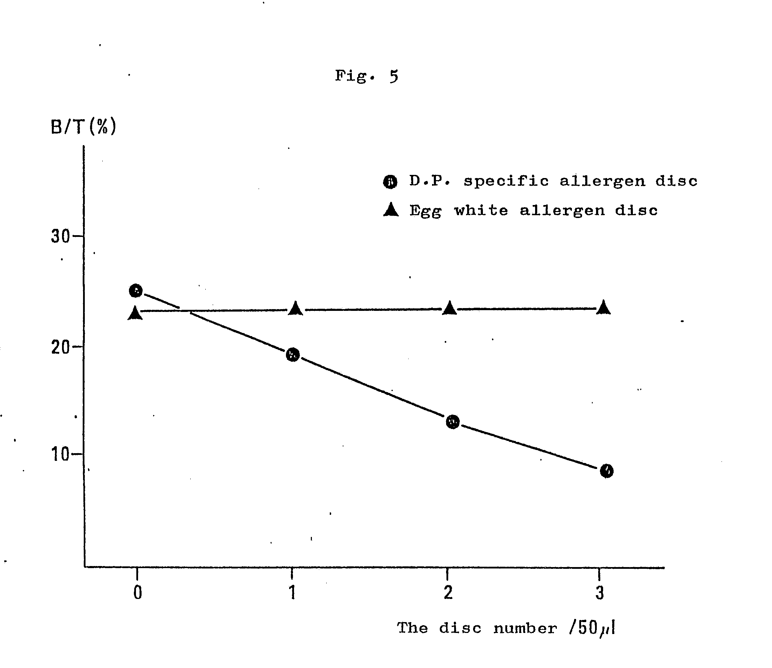

As shown in Fig 5, the D.P. specific IgG antibody is not adsorbed on the egg white allergen disc but on the D.P. allergen disc in proportion to the disc number used. This indicates that the monoclonal antibody has an allergen specificity.

(3) The anti-hIgG used in the present invention very rarely binds to non-specific IgG and are scarcely influenced by non-specific IgG in determining the allergen-specific IgG with a kit of the present invention.

Procedure

The maximum concentration of the specific IgG antibody was fixed to 1000 unit/ml with the aforementioned standard serum, and this was serially diluted. The D.P. specific IgG antibody titer of the corresponding 80 samples was determined. Simultaneously, the total IgG level was determined and the correlation of them was investigated.

Result

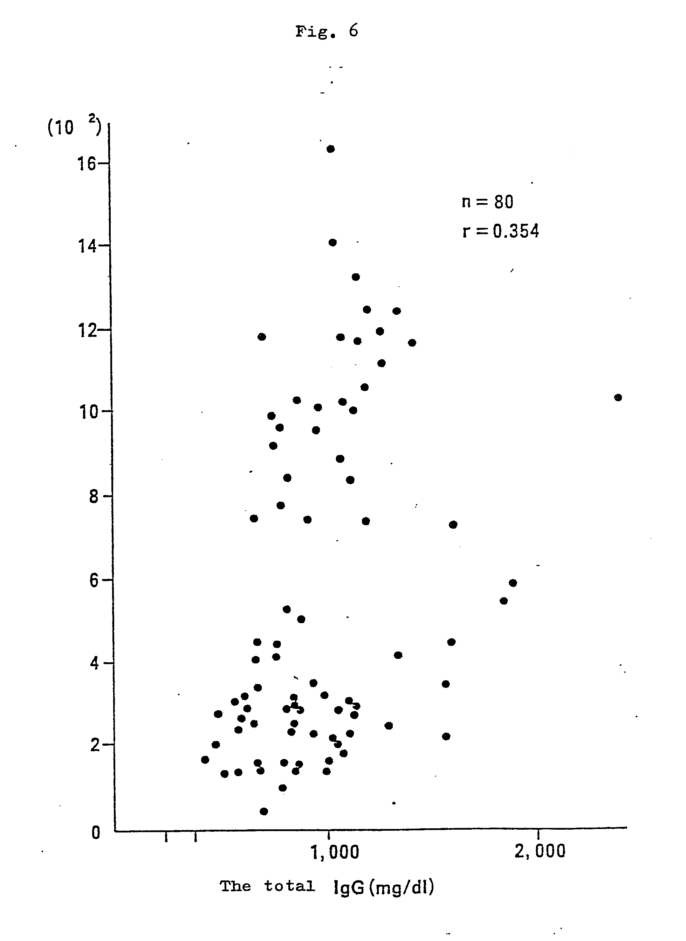

As shown in Fig 6, no relationship is recognized between the titer of D.P. specific IgG antibody and the total IgG level since the correlation coefficient $r$ is 0.354. This means that the influence of non-specific IgG on the data of the specific IgG antibody is extremely rare.

(4) By using the kit of the present invention, the specific IgG antibody can be determined very precisely.

Procedure

Four samples (I to IV) in which the content of D.P. specific IgG antibody is different each other and the reference sera A to F were diluted by a 2-fold dilution method to 50- to 1600-fold concentrations with a serum of atopic allergy patient, and they are allowed to react according to the manner as in Example 3. The absorbance was measured at 415 nm in triplicate, and the relationship between the absorbance and the dilution ratio was plotted on a log-log section paper.

Result

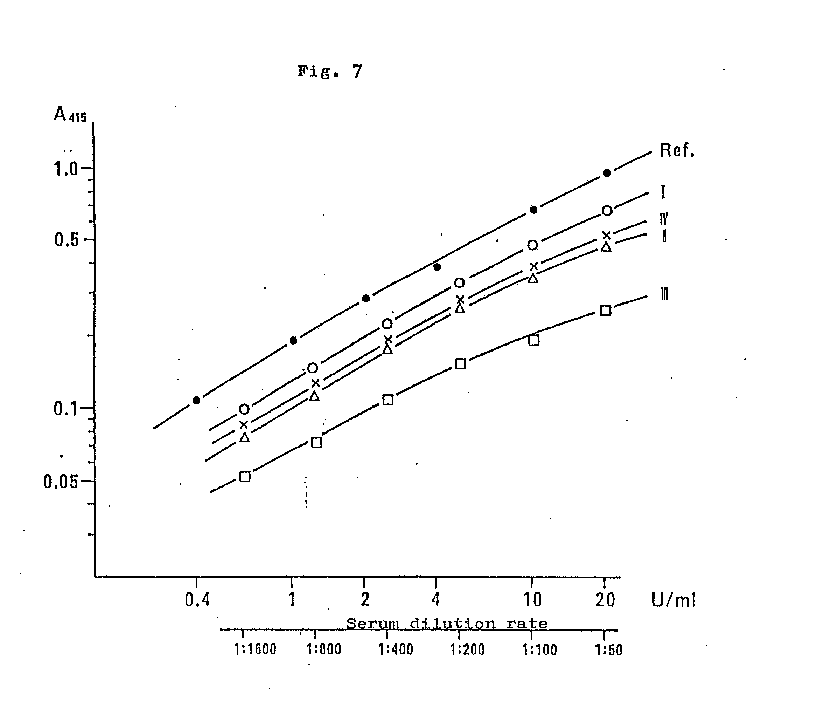

As shown in Fig 7, a linear relation is recognized between the absorbance and the dilution ratio in the reference sera A to F and all of 4 samples. Those lines are parallel each other.

(5) By using the kit of the present invention, the specific IgG antibody can be determined in high sensitivity.

Procedure

Reference serum F (antibody titer: 0.4 U/ml) was diluted to 2 to 64-fold solutions by means of the two-fold dilution method and allowed to react according to the same manner as in Example 3 together with the reference sera C, D, E, and F. The absorbance was measured 5 times at 415 nm and plotted on a semi-logarithmic plotting paper. The same procedure was applied to a diluted solution of NSB (measured 10 times).

Result

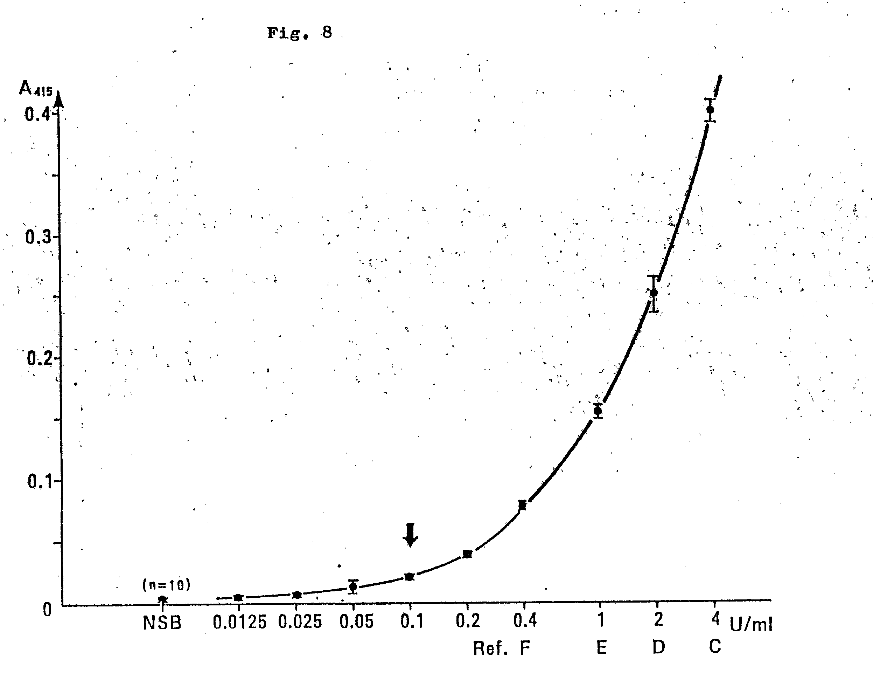

As shown in Fig 8, the sensitivity in measurement corresponds to or is higher than that of 4-fold dilution solution of the reference F (indicated by an arrow); the absorbance of a diluting soltion is very low and quite different from that of the 4-fold dilution solution of the reference F. The C.V. % in the 2-fold and 4-fold dilution solution were 7% or lower.

CLAIMS

(1) Anti-hIgG labelled with a peroxidase.

(2) Enzyme-labelled anti-IgG reagent comprising the following components:

|  |  |  |
|---|---|---|
| (a) | Human serum albumin | 0.06 - 0.2 % |
| (b) | Sodium dihydrogenphosphate | 0.02 - 0.04% |
| (c) | Sodium hydrogendiphosphate | 0.3 - 0.4 % |
| (d) | Sodium chloride | 0.8 - 1.1 % |
| (e) | Anti-hIgG labelled with a peroxidase | 0.01 - 0.02% |
| (f) | Preservative | small amount and |
| (g) | Acid and/or alkali | proper amount |

(3) Specific IgG test reagent comprising the following components:

(a) Enzyme labelled anti-IgG reagent

(b) Colouring agent

(c) Substrate reagent

(d) Reaction terminating agent

(e) Buffering agent

(f) Washing agent and

(g) Diluting agent.

(4) Specific IgG test reagent of Claim 3, in which the colouring agent is ABTS.

(5) Specific IgG test reagent of Claim 3, in which the substrate reagent comprise 0.05-0.6 % of hydrogen peroxide solution and 0.05-0.2 % of phosphoric acid.

(6)  Specific IgG test reagent of Claim 3, in which the reaction terminating reagent is a sodium azide solution.

(7)  Specific IgG test reagent of Claim 3, in which the buffering agent comprises 3.0-8.0 % of citric acid, 5.0-10.0 % of trisodium citrate and a proper amount of stabilizer.

(8)  Specific IgG test reagent of Claim 3, in which the washing agent comprises 1.0-3.0 % of surfactant, 2.0-7.0 % of sodium dihydrogenphosphate, 0.05-3.0 % of alkali metal hydroxide, 15-35 % sodium chloride and a proper amount of stabilizer.

(9)  Specific IgG test reagent of Claim 3, in which the diluting agent comprises 0.05-3.0 % of human serum albumin, 6.0-12.0 % of sodium chloride, 1.0-3.0 % sodium dihydrogenphosphate, 0.1-1.0 % of alkali metal hydroxide and a small amount of preservative.

Fig. 1

Fig. 2

Fig. 3

Fig. 4

Fig. 5

Fig. 6

$(10^2)$

n = 80
r = 0.354

The total IgG (mg/dl)

Fig. 6

Fig. 7

0194496

Fig. 8

0194496